(19) Europäisches Patentamt
European Patent Office
Office européen des brevets

(11) EP 1 185 255 B1

(12) **FASCICULE DE BREVET EUROPEEN**

(45) Date de publication et mention de la délivrance du brevet:
**11.03.2009 Bulletin 2009/11**

(21) Numéro de dépôt: **00951603.0**

(22) Date de dépôt: **16.06.2000**

(51) Int Cl.:
***A61K 31/403*** *(2006.01)* ***A61P 27/02*** *(2006.01)*

(86) Numéro de dépôt international:
**PCT/FR2000/001679**

(87) Numéro de publication internationale:
**WO 2000/076499 (21.12.2000 Gazette 2000/51)**

(54) **MEDICAMENTS OPHTALMOLOGIQUES RETINO-PROTECTEURS comprenant du ramipril ou du ramiprilat**

RETINA PROTEKTIVE OPHTHALMOLOGISCHEN ARZNEIMITTELN enthaltend ramipril oder ramiprilat

RETINOPROTECTIVE OPHTHALMOLOGIC MEDICINES comprising ramipril or ramiprilat

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **16.06.1999 TN 99122**
**06.12.1999 FR 9915359**

(43) Date de publication de la demande:
**13.03.2002 Bulletin 2002/11**

(73) Titulaires:
- **Rekik, Elyes Ben Mohamed Raouf**
**92330 Sceaux (FR)**
- **Rekik, Raouf**
**1002 Tunis (TN)**

(72) Inventeur: **REKIK, Raouf**
**1002 Tunis (TN)**

(74) Mandataire: **Desaix, Anne et al**
**Ernest Gutmann - Yves Plasseraud S.A.S.**
**3, rue Auber**
**75009 Paris (FR)**

(56) Documents cités:

| | |
|---|---|
| **EP-A- 0 114 333** | **EP-A- 0 158 157** |
| **EP-A- 0 220 107** | **WO-A-99/36062** |

- **SHAH, G. B. ET AL: "Ocular hypotensive effect of ramiprilat in chronic and acute models of glaucoma in rabbits" INDIAN J. PHARMACOL. (1999), 31(2), 110-115 , XP000980134**
- **VOGH, BETTY P. ET AL: "Effects of inhibition of angiotensin converting enzyme and carbonic anhydrase on fluid production by ciliary process, choroid plexus, and pancreas" J. OCUL. PHARMACOL. (1989), 5(4), 303-11 , XP000980132**
- **KUSAKA-NAKAMURA, MIHO ET AL: "Antihypertensive treatment in spontaneously hypertensive rats with streptozotocin-induced diabetes mellitus" ACTA PHYSIOL. HUNG. (1988), 71(2), 251-69 , XP000980228**
- **GARG S.K. ET AL: "Renal and retinal changes after treatment with Ramipril and pentoxifyline in subjects with IDDM." ANNALS OF OPHTHALMOLOGY - GLAUCOMA, (1998) 30/1 (33-37). , XP000980142**
- **KULSHRESTHA, M. K. (1) ET AL: "Protective effect of angiotensin converting enzyme inhibitors in diabetic retinopathy." IOVS, (MARCH 15, 1999) VOL. 40, NO. 4, PP. S312. MEETING INFO.: ANNUAL MEETING OF THE ASSOCIATION FOR RESEARCH IN VISION AND OPHTHALMOLOGY FORT LAUDERDALE, FLORIDA, USA MAY 9-14, 1999 ASSOCIATION FOR RESEARCH IN VISION AND OPTHALMOLOGY., XP000980128**
- **YAMAGAMI K. ET AL: "Further study on antihypertensive and antisclerotic effects of captopril on the retinal arteriole in SHRSP." FOLIA OPHTHALMOLOGICA JAPONICA, (1983) 34/2 (290-297). CODEN: NGKYA3, XP000980116**
- **DATABASE WPI Section Ch, Week 199401 Derwent Publications Ltd., London, GB; Class B03, AN 1994-002154 XP002157734 & JP 05 306296 A (ZH MARINO FORUM 21), 19 novembre 1993 (1993-11-19)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

**(Cont. page suivante)**

- **DETRY-MOREL M: "PERSPECTIVES DANS LE TRAITEMENT MEDICAL DE LA NEUROPATHIE GLAUCOMATEUSE. BASES DE LA NEUROPROTECTION" JOURNAL FRANCAIS D'OPHTALMOLOGIE,FR,MASSON, PARIS, vol. 22, no. 1, 1999, pages 122-134, XP000870144 ISSN: 0181-5512 cité dans la demande**
- **"Merck Manual of diagnosis and therapy" 1999, MERCK RESEARCH LABORATORIES , WHITEHOUSE STATION N.J., USA * page 1641 ***
- **SHAH ET AL: 'Oculohypotensive effect of angiotensin-converting enzyme inhibitors in acute and chronics models of glaucoma' JOURNAL OF CARDIOVASCULAR PHARMACOLOGY vol. 36, no. 2, Août 2000, pages 169 - 175**

## Description

L'invention concerne la production de médicaments rétinoprotecteurs pour maintenir ou améliorer la fonction visuelle (acuité et champ visuels). Elle concerne plus particulièrement de tels médicaments pour le traitement d'affections ophtalmologiques comportant une détérioration de la chorio-rétine) avec pour conséquence une perte progressive de la vision (acuité et champ visuels).

Certains documents antérieurs ont déjà évalué l'action présumée des inhibiteurs de l'enzyme de conversion (IEC) de l'angiotensine sur la pression oculaire (PIO). Ils n'ont pas relevé leur intérêt dans l'amélioration de la vision. Par ailleurs article récent de M. Détry Morel paru dans la revue J. Fr. Ophtalmol., 1999 ; 22,1, 122-134 (1999), intitulé « Perspectives dans le traitement médical de la neuropathie glaucomateuse - Bases de la neuroprotection », qui passe en revue les perspectives à ce jour du développement de médicaments pour le traitement médical des neuropathies glaucomateuses fait référence à l'utilisation qui aurait été envisagée de quelques médications vasoactives mettant en jeu certains inhibiteurs de l'angiotensine de conversion pour surmonter l'amoindrissement de la circulation sanguine oculaire ("ocular blood flow") qui est observable chez des patients porteurs de certaines formes de glaucomes. Mais, selon Détry-Morel, si certaines de ces médications étaient susceptibles d'induire un accroissement de cet « ocular blood flow » il conviendrait toutefois d'être très prudent puisque l'on ne sait pas si cet accroissement s'accompagne d'un effet bénéfique pour les patients ou, au contraire, consiste seulement en un effet secondaire. Détry-Morel évoque en particulier la possibilité que cet accroissement, normalement bien géré par un tissu sain, puisse avoir des conséquences désastreuses dans un tissu dont le lit vasculaire serait au départ précaire. Enfin, Détry-Morel ne rapporte aucune observation de l'interruption du processus de détérioration du nerf optique ou de la perte progressive du champ visuel, encore moins d'une amélioration du champ visuel, quels que soient les traitements qu'il a passés en revue.

EP0220107 divulgue l'utilisation des IEC pour le traitement de la dégénérescence maculaire liée à l'âge.

Shah et al. (indian journal of pharmacology 1999; 31: 110-115) divulgue un effet hypotenseur oculaire du ramiprilat administré par voie topique dans un modèle de glaucome chez le lapin. EP0158157 et EP0114333 décrivent l'utilisation d'IEC pour abaisser la tension intraoculaire et pour le traitement du glaucome.

Kusaka-Nakamura et al. (Acta Physiologica Hungarica, 1988, 71(2) 251-269) décrit que le ramipril administré par voie orale permet de normaliser la tension et d'empêcher la progression des lésions de la rétine chez des rats diabétiques et hypertendus.

Garg et al. (Ann. Ophthalmol. 1998; 30(1) 33-37) divulgue l'utilisation du ramipril pour le traitement de la rétinopathie chez des patients diabétiques.

Kulshrestha et al. (IOVS, 03.1999, 40(4), 1652-B560) divulgue l'utilisation des IEC pour empêcher la progression de la rétinopathie diabétique chez des patients diabétiques et hypertendus.

Yamagami et al. (Folia Ophthalmologica, 1983, 34(2) 290-297) étudie l'effet antihypertenseur du captopril.

Le résumé WPI du brevet japonais JP05306296 divulgue un peptide ayant un effet IEC pour le traitement des occlusions rétinales véneuses.

L'invention a pour but la production de médicaments destinés au traitement des affections ophtalmologiques permettant l'obtention d'une amélioration des fonctions visuelles, en particulier du champ visuel et de l'acuité visuelle, chez des patients souffrant d'affections chorio-rétiniènes, qui font intervenir un facteur vasculaire. Au nombre de ces pathologies on mentionnera,

(1) la chorio-rétinopathie dégénérative du myope fort,
(2) les épithéliopathies rétiniennes diffuses (RD) et la choriorétinopathie séreuse centrale (C.R.S.C.),
(3) les dystrophies héréditaires de la rétine.

L''invention découle de la découverte que certains inhibiteurs de l'enzyme de conversion (IEC) de l'angiotensine 1 en angiotensine 2 avaient pour effet d'induire chez les patients traités non seulement une interruption, ce que l'on n'avait guère observé à ce jour chez de patients traités pour des affections du type sus-indiqué, mais de surcroît une inversion au moins partielle du processus de dégradation de la fonction visuelle avec amélioration de l'acuité visuelle et du champ visuel.

Mais les médicaments selon l'invention ne sont pas limités aux applications qui ont été évoquées ci-dessus. Ils sont utilement mis en oeuvre pour prévenir ou ralentir, voire supprimer, les diminutions « naturelles » de l'acuité visuelle du champ visuel ou des deux à la fois. En particulier, ils trouvent une application heureuse dans la prévention et même l'inversion du déclin visuel chez le sujet âgé.

La sensibilité rétinienne moyenne diminue de façon linéaire avec l'âge. Cette pente s'amorce très tôt, au moins dès l'âge de 20 ans et s'accélère après 60 ans. En particulier la sensibilité moyenne (SM) exprimée en décibels (dB) obéit pour Jaffe à l'équation suivante :

$$SM (dB) = 28.8 - 0.074 \times \text{âge}.$$

Ceci est d'ailleurs utilisé par l'octopie pour le calcul de la sensibilité corrigée pour l'âge. Dans les 30° centraux, la sensibilité rétinienne décline non seulement plus vite en périphérie qu'au centre mais aussi plus en périphérie supérieure que dans les autres quadrants.

L'administration d'un IEC approprié, en particulier de ramipril aux sujets normaux a montré, périmétrie automatique à l'appui, une amélioration des sensibilités moyennes excédant de 1 à 2 DB, les valeurs de sensibilité moyenne considérées comme normales par les logiciels d'étude, puisqu'ils intègrent même dans l'évaluation de la normalité de ces sensibilités moyennes les pertes « normales » dont elles sont l'objet en fonction de l'âge des sujets chez lesquels on cherche à déterminer ce que sont les pertes en fait supplémentaires de sensibilité moyenne, dont ils souffrent lorsqu'ils sont affectés, par exemple par l'une des pathologies sus-mentionées.

La molécule à ce jour la plus efficace est constituée par le ramipril, de formule :

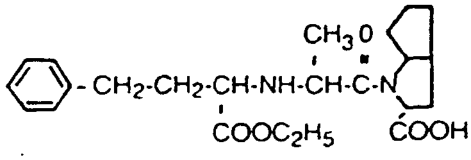

ou le ramiprilate, qui résulte de la désestérification du ramipril, de formule :

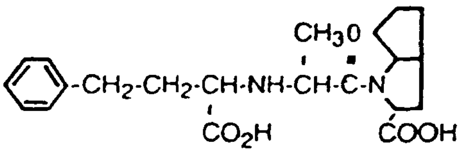

Sans doute ces médicaments font-ils intervenir le mécanisme d'action des IEC, dans la mesure où ils agissent en empêchant la transformation de l'angiotensine I en angiotensine II (vasoconstricteur) et la dégradation des bradykinines (vasodilatateurs). Ces IEC entraînent donc une vasodilatation, qui s'exerce aussi bien sur les artères que sur les veines. On parle de vasodilatateurs mixtes. Rappelons que le mécanisme de formation de la liaison qui peut s'établir entre l'inhibiteur et l'enzyme se déroule normalement en deux phases, la première consistant en la formation d'un complexe inhibiteur-enzyme par une liaison compétitive, qui met en jeu une réaction régie par une constante $K_i$ et la seconde en une isomérisation lente du complexe inhibiteur-enzyme, elle-même également régie par une constante $k_4$ de vitesse de réversibilité de cette réaction.

Il peut être fait référence, pour ce qui est de l'explicitation de ces concepts, à la publication de D. Vasmant et N. Bender intitulée « Système rénine-angiotensine et ramipril, un nouvel inhibiteur de l'enzyme de conversion » dans journal of cardiovascular Pathology 14 (suppl. 4) (1989), : S49-S56, Raven Press, qui renvoie d'ailleurs aussi à des publications antérieures de Bünning P. « Inhibition of angiotensin converting enzyme by 2-(N-((S)-1-carboxy-3-phenoxy)-L-alanyl)-(1S,3S,5S)-2-azabicyclo (3.3.0)octane-3-carboxylic acid (HOE A98 diacid. Comparison with captopril and enalaprilat" dans Drug Research 1984 ;34 :1406-1410, ainsi qu'à la these de doctorat de Shapiro R. (Ph.D Thesis, Harvard University Cambridge. MA, 1983 "Activation and inactivation of rabbit pulmonary angiotensin converting enzyme".

Le ramipril et le ramiprilate sont d'un intérêt particulier. Le ramiprilate, qui résulte de la désestérification du ramipril, peut former des complexes plus stables que d'autres inhibiteurs avec l'enzyme de conversion de l'angiotensine. En particulier, la demi-vie de ces complexes est de l'ordre de 12 à 17 heures. Ils se distinguent aussi de nombreux autres inhibiteurs de l'enzyme de conversion de l'angiotensine par leur lipophilie et par la stabilité plus élevée des couples enzyme-inhibiteur formés. Toutes ces propriétés paraissent concourir à la manifestation des effets qu'ils permettent d'obtenir à très faibles doses, comme cela est illustré par les exemples fournis plus loin.

Il est en effet souhaitable que les doses efficaces pour obtenir les effets dans le domaine de l'ophtalmologie soient inférieures aux doses qui induiraient une hypotension artérielle générale chez un sujet ayant une tension normale. Il va de soi naturellement que les doses utilisables peuvent être plus élevées chez des sujets hypertendus, sans que cela soit pour autant nécessaire, pour ce qui est de l'effet recherché dans le domaine ophtalmologique.

L'invention concerne donc des médicaments rétino-protecteurs mettant en jeu, à titre de principe actif le ramipril, le ramiprilat, ou l'un de leur sels. Ces composés sont susceptibles de former avec l'enzyme de conversion de l'angiotensine un complexe suffisamment stable permettant d'obtenir non seulement l'interruption du processus de la dégradation de la fonction visuelle chez les patients qui souffrent des pathologies en cause, mais en outre une inversion ou régression de ce processus.

En particulier, ils présentent simultanément

une constante d'inhibition $K_i$ d'équilibre qui régit la réaction d'inhibition in vitro de l'enzyme de conversion de lapin par l'IEC en cause inférieure à celle de l'énaprilate(50 pmol/L), et

une constante $k_4$ de réversibilité de la réaction d'isomérisation du complexe enzyme-inhibiteur formé inférieure à celle de l'énalaprilate ($1{,}1 \times 10^{-4}$.)(S1) dans un milieu (50) (mM/L Hepes, 300 mmol/L NaCl, I micromol/L ZnCl2, pH 7,5 selon les méthodes de Bünning et de Shapiro dans les conditions décrites par ces auteurs dans les articles identifiés plus haut.

Avantageusement encore ils présentent une lipophilie supérieure à celle de l'énalaprilate, par exemple dans des conditions mettant en jeu des mesures comparatives de coefficients de partage dans un milieu octanol/eau à pH acide. La plus grande lipophilie se manifestera par un passage d'une proportion de l'IEC selon l'invention dans la phase octanol plus importante que pour l'enalaprilate dans une plage substantielle de pHs acides notamment dans l'intervalle de pH 2 - 4.

L'énalaprilate présente déjà, lorsqu'on l'utilise en lieu et place du ramipril, une amélioration modérée de la fonction visuelle chez les patients traités. Mais les effets observés avec le ramiprilate dont la constante $K_i$ est de 7 (7 fois plus forte qu'avec l'énalaprilate) et la constante $k_4$ de $1{,}8 \times 10^{-5}$ (de sorte que le complexe que forme le ramiprilate avec l'enzyme est six fois plus stable que celui que forme l'énalaprilate) sont incomparablement supérieurs. Il est rappelé que les indications de nature physico-chimique qui précèdent sont issues de l'article de Vasmant et al, *loc. cit.*

Des observations semblables fondées sur des essais réels n'ont pas seulement été faites chez des personnes atteintes de neuropathies glaucomateuses, mais également chez des personnes souffrant de tous autres types d'affections mettant en jeu un facteur vasculaire.

Plus particulièrement l'invention concerne l'utilisation des composés sus-indiqués pour la fabrication de médicaments utilisables pour induire chez les personnes traitées une amélioration de l'acuité et du champ visuel.

L'invention concerne encore les compositions pharmaceutiques dans lesquelles les susdits principes actifs sont associés à des véhicules pharmaceutiquement acceptables permettant leur administration sous différentes formes, notamment : formes orale, parentérale, intraveineuse, intramusculaire et transdermale, et formes topiques, notamment sous forme de collyres.

Sans que les indications chiffrées qui suivent soient à considérer comme ayant un caractère restrictif, tant elles relèvent du clinicien et de son malade, les doses quotidiennes suivantes de principe actif - à savoir le ramipril ou le ramiprilate - sont efficaces, à raison de 0,5 à 5, de préférence 1 à 2 mg/jour, par exemple 1,25 mg/jour, lorsqu'elles sont administrées par voie orale. Il est clair que la posologie peut varier d'un patient à l'autre et qu'elle relève *in fine* du clinicien. Ces doses peuvent être différentes, lorsque l'on à recours à des médicaments distincts.

Une préférence nette est marquée pour les formes d'administration topiques, notamment sous forme de collyres. Elles s'avèrent particulièrement efficaces. Elles viennent en effet combler un vide dans le traitement des affections ophtalmologiques qui comportent un facteur vasculaire et que l'on ne traitait pas de façon efficace jusqu'à ce jour.

En d'autres termes, le namipril, le ramiprilate ou l'un de leurs sels sont, dans des formes préférées du médicament selon l'inventeur, associés à des véhicules pharmaceutiques permettant leur application en clinique sous formes de collyres.

L'invention sera encore davantage illustrée par la description des exemples cliniques qui suivent et qui, bien entendu, n'ont aucun caractère limitatif. Dans ces exemples le principe actif, en particulier le ramipril, a été administré en continu sous forme orale, à raison de 1,25 mg par jour.

Il est remarquable que des observations équivalentes sont faites chez des sujets « normaux », en particulier vieillissants, auxquels sont administrés le médicament, ces sujets vieillissants retrouvant alors des sensibilités moyennes qu'ils avaient connues, lorsqu'ils étaient beaucoup plus jeunes.

<u>**NEUROPATHIE GLAUCOMATEUSE**</u> (exemple comparatif)

L'origine ischémique de la neuropathie gaucomateuse a été soupçonnée au vu de l'exploration angiographique. L'angiographie fluoresceinique a montré :

**1)** <u>Papille</u> :

- soit un retard d'imprégnation de la papille glaucomateuse ;
- soit des défauts d'imprégnation absolus. Ces déficits sont en corrélation avec les altérations du champ visuel.

**2)** Choroïde :
L'angiographie fluorescéinique a montré :

- un retard de remplissage choroïdien : dans 100% des cas ;
- une irrégularité de remplissage en secteurs ;
- une visibilité anormale des vaisseaux choroïdiens principaux.

**3)** Rétine :

- Temps artériel rétinien plus long.

Les patients porteurs de glaucome connu et présentant une hypertension artérielle ont vu leur acuité visuelle ainsi que leur champ visuel s'améliorer en très peu de temps, dès que le traitement a été institué. On soulignera parmi les effets obtenus :

- Acuité Visuelle : amélioration nette, le cas le plus spectaculaire est celui d'un patient dont l'acuité visuelle est passée de 3/10 à 10/10 (OD) et de 6/10à8/10(OG);
- Champ visuel : Etudié au goldmann et surtout au périmètre automatique s'est amélioré de façon nette et spectaculaire ; en moyenne la perte de la sensibilité moyenne a baissé de 30%, ce qui est original et exceptionnel ;
- Tension oculaire : baisse significative mais dont l'importance est irrégulière.

La baisse tout à fait relative de la pression intraoculaire n'explique pas des améliorations aussi rapides et spectaculaires du champ visuel. Sous ramipril, la perte de la sensibilité moyenne au périmètre automatique (octopus) a baissé en moyenne de 30%, des scotomes absolus sont devenus relatifs. Si l'élévation de la pression intra-oculaire constitue un facteur aggravant de la neuropathie glaucomateuse, cette dernière apparaît comme consistant essentiellement en une maladie vasculaire ischémique nécessitant un traitement à visée vasculaire.

Les figures 1 à 4 attestent également de l'obtention de l'amélioration de la fonction visuelle, en particulier dans les conditions qui ont été rappelées plus haut. Les données chiffrées liées à ces figures apparaissent également dans les tableaux ci-après (dont les numéros correspondent naturellement à ceux des figures) :

**Tableau 1**

| Oeil gauche avant traitement | | | | | | |
|---|---|---|---|---|---|---|
| | | | **Normal** | **Phase 1** | **Phase 2** | **Moyenne** |
| Sensibilité moyenne | Ms | | | 16.9 | 17.3 | 16.5 |
| Perte moyenne | MD | [dB] | -2..2 | 9.5 | 9.4 | 9.9 |
| Variance de la perte | LV | [dB] | 0..6 | 26.8 | 29.3 | |
| Variance de la perte corr. | CLV | [dB]$^2$ | 0..4 | | | 22.4 |
| Fluctuation à court terme | SF | [dB]$^2$ | 0..2 | | | 2.7 |
| Indice de fiabilité | RF | [%] | | | | 4.9 |

**Tableau 2**

| Oeil gauche après traitement | | | | | | |
|---|---|---|---|---|---|---|
| | | | **Normal** | **Phase 1** | **Phase 2** | **Moyenne** |
| Sensibilité moyenne | MS | | | 20.0 | 20.0 | 20.2 |
| Perte moyenne | MD | [dB] | -2..2 | 5.7 | 6.8 | 6.2 |
| Variance de la perte | LV | [dB] | 0..6 | 16.8 | 26.5 | |
| Variance de la perte corr | CLV | [dB]$^-$ | 0..4 | | | 13.1 |

(suite)

| Oeil gauche après traitement | | | | | | |
|---|---|---|---|---|---|---|
| | | | Normal | Phase 1 | Phase 2 | Moyenne |
| Fluctuation à court terme | SF | [dB]$^-$ | 0..2 | | | 3.0 |
| Indice de fiabilité | RF | [%] | | | | 5.0 |

**Tableau 3**

| Oeil droit avant traitement | | | | | | |
|---|---|---|---|---|---|---|
| | | | Normal | Phase 1 | Phase 2 | Moyenne |
| Sensibilité moyenne | MS | | | 17.4 | 17.4 | 16.9 |
| Perte moyenne | MD | [dB] | -2..2 | 9.0 | 9.3 | 9.5 |
| Variance de la perte | LV | [dB] | 0..6 | 28.6 | 21.0 | |
| Variance de la perte corr | CLV | [dB]$^-$ | 0..4 | | | 21.8 |
| Fluctuation à court terme | SF | [dB]$^-$ | 0..2 | | | 3.0 |
| Indice de fiabilité | RF | [%] | | | | 2.3 |

**Tableau 4**

| Oeil droit après traitement | | | | | | |
|---|---|---|---|---|---|---|
| Normal | | | | Phase 1 | Phase 2 | Moyenne |
| Sensibilité moyenne | MS | | | 22.2 | 21.9 | 21.6 |
| Perte moyenne | MD | [dB] | -2..2 | 4.1 | 4.7 | 4.8 |
| Variance de la perte | LV | [dB] | 0..6 | 17.2 | 10.4 | |
| Variance de la perte corr. | CLV | [dB]$^2$ | 0..4 | | | 13.1 |
| Fluctuation à court terme | SF | [dB]$^2$ | 0..2 | | | 2.6 |
| Indice de fiabilité | RF | [%] | | | | 2.3 |

Il découle déjà des figures une amélioration nette de la fonction visuelle ; Les tableaux en fournissent une illustration complémentaire, en particulier au plan de la sensibilité moyenne qui passe de 16,5 à 20,2 pour l'oeil gauche, et de 16,9 à 21,6 pour l'oeil droit. De même on note une forte régression de la perte moyenne de vision par rapport à la normale ; de 9,9 à 6,2 pour l'oeil gauche et de 9,5 à 4,8 pour l'oeil droit.

## CHORIO-RÉTINOPATHIE DÉGÉNÉRATIVE DU MYOPE FORT

L'hypothèse d'un facteur vasculaire dans la genèse des lésions choroïdiennes est avancée, du fait de la présence de lésions vasculaires de la choroïde. L'occlusion vasculaire touche d'abord la choriocapillaire qui est atrophique, puis intéresse les vaisseaux de moyen puis de gros calibre. Il s'ensuit une atrophie et même une disparition de la choroïde prédominant au pole postérieur et conditionnant au moins en partie l'atteinte rétinienne qui devient atrophique. Néanmoins, les vaisseaux rétiniens participent à cette atrophie. L'amélioration de l'acuité visuelle corrigée ne semble pas, à ce jour, avoir jamais été constatée chez des patients très myopes et affectés par une chorio-rétinopathie dégénérative.

Même à faible dose, le ramipril entraîne toujours une amélioration de l'acuité visuelle corrigée et cela malgré l'existence d'une choroïdose myopique extrêmement sévère. A titre d'exemples, l'acuité visuelle est passée respectivement : de la simple perception lumineuse (PL) à 1/20 sur oeil monophtalme ; de 2/10 (OD) 1/10 (OG) à 5/10 (OD) et 5/10 (OG) ; de 1,5/10 à 3/10 ; de 1/20 (2 yeux) à 2/10 (OD) et 1/10 (OG) ; de 1/10 (OD) et 1/10 (OG) à 6/10 (OD) et 3/10 (OG). Il est entendu que dans ce qui précède OD signifie "oeil droit" et OG "oeil gauche".

**DÉGÉNÉRESCENCE MACULAIRE LIÉE À L'ÂGE (D.M.L.A.)** (exemple comparatif)

Le caractère ischémique de la D.M.L.A. a été évoqué sur la base des données suivantes.

**Données angiographiques :**

Un travail récent aurait mis en évidence un ralentissement de la perfusion choroïdienne maculaire chez 26 sur 100 patients présentant une D.M.L.A.

**Etude histologique :**

Elle a montré :

- une augmentation progressive du tissu intervasculaire qui peut rétrécir la lumière de la chorio-capillaire et des artérioles afférentes ;
- une diminution du nombre et du diamètre des capillaires choroïdiens ;
- une diminution du nombre des vaisseaux choroïdiens moyens ;
- des anomalies, notamment une atrophie du lit capillaire rétinien périfovéolaire : il paraît atrophique.

La plupart des patients présentant une dégénérescence maculaire liée à l'âge avec ou sans neovaisseaux sous-rétiniens traités par le ramipril ont vu leur acuité visuelle s'améliorer. A titre d'exemple, l'acuité visuelle est passée respectivement de 0,16 à 0,3 ; 0,2 à 0,5 ; de 0,05 à 0,2 (l'oeil droit) et 0,05 à 0,15 (l'oeil gauche) ; de 0,2 à 0,4 (l'oeil droit) et de 0,5 à 0,8 (l'oeil gauche) ; de 0,6 à 1 ; de 0,4 à 0,6 (l'oeil droit) et 0,2 à 0,5 l'oeil gauche).

**DYSTROPHIES HEREDITAIRES DE LA RETINE ET DE L'EPITHELIUM PIGMENTAIRE**

**Rétinite pigmentaire :**

Cette affection correspond à une dystrophie des cônes et des bâtonnets. Elle est caractérisée par la survenue d'une cécité nocturne dans l'enfance ou l'adolescence, un rétrécissement progressif du champ visuel périphérique débutant par un scotome annulaire équatorial et qui évolue vers une baisse de l'acuité visuelle importante ou même une cécité à l'âge adulte.

**Une composante vasculaire** a été démontrée dans ce type de dystrophie. Elle est étayée par la clinique, l'angiographie et l'histologie :

1. Clinique : diminution du calibre des vaisseaux rétiniens.
2. Angiographie :
   - augmentation du temps de circulation rétinienne,
   - dilatation des capillaires rétiniens et papillaires,
   - retard circulatoire choroïdien.
3. Histopathologie :
   - épaississement avec hyalisation des parois des vaisseaux rétiniens,
   - oblitération partielle de la chorio-capillaire qui devient atrophique.

**EVOLUTION ET PRONOSTIC**

Le pronostic est sévère. Il est marqué par une progression constante de l'affection vers la cécité. Il n'existe pas de traitement connu.

De l'existence de ce facteur vasculaire est venue l'idée de l'essai des médications selon l'invention dans les dystrophies héréditaires de la rétine et de l'épithélium pigmentaire.

Le ramipril a été utilisé dans les trois affections suivantes : rétinite pigmentaire ; maladie de stargardt et fundus flavimaculatus ; dystrophie pseudo-vitelliforme de l'adulte. Il a entraîné dans tous les cas une amélioration de la fonction visuelle.

Rétinite Pigmentaire

Trois patients ont été traités au ramipril 1.25 mg par voie orale et ont vu leur acuité visuelle s'améliorer en l'espace de deux mois :

**Premier patient :** l'acuité visuelle est passée respectivement à l'oeil droit de perception lumineuse douteuse à 1/50 et à l'oeil gauche de 1/50 à 1.6/10.
**Deuxième patient :** Monophtalme l'acuité visuelle est passée de 2/10 à 5/10.
**Troisième Patient :** Souffrant d'une rétinite pigmentaire sectorielle, il a vu son acuité visuelle passer de 1.6 à 6/10 à droite et de 1.6 à 4/10 à gauche. La sensibilité moyenne au champ visuel automatique octopus s'est amélioré de 16,5 à 18 à droite et de 16.8 à 19.9 à gauche.

L'électro-rétinogramme s'est sensiblement amélioré surtout au rouge et à l'oeil gauche.

Maladie de stargardt flavimaculatus

Un patient a été traité, son acuité s'est amélioré de 1/20 à 1/10 à droite et de 1/50 à 3/10 à gauche.

Dystrophie pseudovitelliforme

Un patient a été testé. Son acuité est passée en un mois de 1.6 à 2/10 à droite et 2/10 à 3/10 à gauche. L'activité du ramipril dans ces dystrophies héréditaires est unique.
Des reproductions des champs visuels appréciés chez le troisième patient porteur de rétinite pigmentaire sectorielle, respectivement avant et après deux mois de traitement, sont, à titre d'exemple, montrées dans les figures 5 et 6 pour l'oeil gauche, dans les figures 7 et 8 pour l'oeil droit.
Des figures 5 à 8 découlent également une amélioration substantielle de la fonction visuelle. Les données chiffrées des tableaux 5 à 8 ci-après qui respectivement leur correspondent, en fournissent une illustration supplémentaire. En particulier la sensibilité moyenne passe de 16,8 à 20,9 pour l'oeil gauche et de 16,5 à 18,3 pour l'oeil droit. De même la perte moyenne de vision par rapport à la normale régresse de 10,3 à 6,5 pour l'oeil gauche et de 10,6 à 8,8 pour l'oeil droit.

**Tableau 5**

| Oeil gauche avant traitement | | | | | | |
|---|---|---|---|---|---|---|
| | | | **Normal** | **Phase 1** | **Phase 2** | **Moyenne** |
| Sensibilité moyenne | MS | | | 17.3 | 17.9 | 16.8 |
| Perte moyenne | MD | [dB] | -2..2 | 9.8 | 9.6 | 10.3 |
| Variance de la perte | LV | [dB] | 0..6 | 60.6 | 52.8 | |
| Variance de la perte corr. | CLV | $[dB]^2$ | 0..4 | | | 52.5 |
| Fluctuation à court terme | SF | $[dB]^2$ | 0..2 | | | 2.5 |
| Indice de fiabilité | RF | [%] | | | | 9.8 |

**Tableau 6**

| Oeil gauche après traitement | | | | | | |
|---|---|---|---|---|---|---|
| | | | **Normal** | **Phase 1** | **Phase 2** | **Moyenne** |
| Sensibilité moyenne | MS | | | 21.2 | 20.8 | 20.9 |
| Perte moyenne | MD | [dB] | -2..2 | 6.2 | 7.0 | 6.5 |
| Variance de la perte | LV | [dB] | 0..6 | 51.8 | 63.9 | |
| Variance de la perte corr. | CLV | $[dB]^2$ | 0..4 | | | 48.0 |
| Fluctuation à court terme | SF | $[dB]^2$ | 0..2 | | | 1.9 |

(suite)

| Oeil gauche après traitement | | | | | | |
|---|---|---|---|---|---|---|
| | | | **Normal** | **Phase 1** | **Phase 2** | **Moyenne** |
| Indice de fiabilité | RF | [%] | | | | 7.9 |

**Tableau 7**

| Oeil droit avant traitement | | | | | | |
|---|---|---|---|---|---|---|
| | | | **Normal** | **Phase 1** | **Phase 2** | **Moyenne** |
| Sensibilité moyenne | MS | | | 16.8 | 16.0 | 16.5 |
| Perte moyenne | MD | [dB] | -2..2 | 10.2 | 11.5 | 10.6 |
| Variance de la perte | LV | [dB] | 0..6 | 70.4 | 86.9 | |
| Variance de la perte corr. | CLV | $[dB]^2$ | 0..4 | | | 66.0 |
| Fluctuation à court terme | SF | $[dB]^2$ | 0..2 | | | 2.4 |
| Indice de fiabilité | RF | [%] | | | | 12.2 |

**Tableau 8**

| Oeil droit après traitement | | | | | | |
|---|---|---|---|---|---|---|
| | | | **Normal** | **Phase 1** | **Phase 2** | **Moyenne** |
| Sensibilité moyenne | MS | | | 18.4 | 18.2 | 18.3 |
| Perte moyenne | MD | [dB] | -2..2 | 8.7 | 9.2 | 8.8 |
| Variance de la perte | LV | [dB] | 0..6 | 67.5 | 79.8 | |
| Variance de la perte corr. | CLV | $[dB]^2$ | 0..4 | | | 65.6 |
| Fluctuation à court terme | SF | $[dB]^2$ | 0..2 | | | 1.7 |
| Indice de fiabilité | RF | [%] | | | | 10.3 |

**OCCLUSIONS VEINEUSES RETINIENNES** (exemple comparatif)

Le syndrome d'occlusion de la veine centrale de la rétine ou de l'une de ses branches correspond à un ralentissement plus ou moins important du courant circulatoire. Il n'existe pas d'arrêt complet.

Il est défini par la présence de symptômes fondamentaux :

- altération de la vision,
- retard circulatoire rétinien,
- dilatation veineuse,
- hémorragies rétiniennes,
- oedème rétinien diffus,
- nodules cotonneux,
- altération du lit capillaire.

Le pronostic de ce type d'affection est incertain. Il est fonction de la forme clinique. Des complications peuvent aggraver la situation, en particulier l'oedème maculaire, l'hémorragie du vitre et le glaucome néovasculaire.

Le seul traitement connu est la photocoagulation au laser. Ce dernier traite les complications et non l'occlusion elle-même.

La plupart des patients traités au ramipril se sont améliorés, aussi bien sur le plan fonctionnel qu'angiographique.

A titre d'exemple, deux patients présentant une occlusion de branche veineuse de type oedémateux et ischémique ont vu leur acuité visuelle s'améliorer respectivement de 2/10 à 8/10 et de 6/10 à 10/10 et cela en l'espace de

deux mois.

Le ramipril en améliorant la pression de perfusion augmente le débit (blood flow) et traite ainsi directement l'occlusion veineuse qui se définit encore une fois comme un ralentissement circulatoire veineux.

## ÉPITHÉLIOPATHIE RÉTINIENNE ET CHORIO-RÉTINOPATHIE SÉREUSE CENTRALE (C.R.S.C.)

Des altérations vasculaires ont été incriminées. L'angiographie au vert d'indocyanine a montré ces altérations vasculaires choroïdiennes diffuses avec hyper-perméabilité choroïdienne.

Cette dernière entraîne un décollement séreux de l'épithélium pigmentaire. La pression exercée sur l'épithélium pigmentaire produit une fuite qui, de façon mécanique, occasionne un décollement séreux rétinien maculaire.

L'utilisation du ramipril a entraîné en quelques jours à quelques semaines un affaissement de la bulle et donc une application du neuro-epithélium rétinien : ce qui a conduit à une amélioration de l'acuité visuelle ainsi que du champ visuel central

Pour le ramipril et le ramiprilate, l'amélioration de la fonction visuelle se manifeste à des doses n'induisant pas d'hypotension artérielle sur le sujet sain.

Compte tenu de l'action vasodilatatrice du ramipril et du ramiprilate et de la part de responsabilité du facteur vasculaire dans le développement des six pathologies que sont la neuropathie glaucomateuse, la choriorétinopathie dégénérative du myope fort, la D.M.L.A., la choriorétinopathie séreuse centrale, l'occlusion veineuse rétinienne et les dystrophies héréditaires de la rétine, on comprend que ces substances ne peuvent qu'avoir un effet dans toutes autres affections choriorétiniennes comportant une composante vasculaire.

Il est entendu que l'expression "fonction visuelle", telle qu'elle est utilisée dans les revendications qui suivent, fait référence plus particulièrement, sans pour autant y être limité, à l'acuité visuelle ou au champ visuel ou, de préférence, aux deux à la fois.

L'invention concerne encore un médicament ophtalmologique rétino-protecteur, apte à induire une interruption du processus de dégradation de la fonction visuelle et même à en inverser le cours, caractérisé par le fait que son principe actif est constitué par un inhibiteur de l'enzyme de conversion de l'angiotensine 1 en angiotensine 2, choisi parmi le ramipril, le ramiprilate ou l'un de leurs sels ayant un grand degré de stabilité, étant encore précisé que l'inhibiteur présente, simultanément :

- une constante d'inhibition $K_i$ d'équilibre qui régit la réaction d'inhibition in vitro de l'enzyme de conversion de lapin par l'inhibiteur inférieure à celle de l'énalaprilate(50 pmol/L), et

- une constante $k_4$ de réversibilité de la réaction d'isomérisation du complexe enzyme-inhibiteur formé inférieure à celle du complexe formé par l'énalaprilate et l'enzyme de conversion (1,1 x $10^{-4}$.)(S1) dans un milieu (50) (mM/L Hepes, 300 mmol/L NaCl, I micromol/L ZnCl2, pH 7,5. dont l'activité vasodilatatrice se manifeste au niveau non seulement des artères, mais aussi des veines.

L'invention concerne encore un médicament dans lequel le ramipril ou le ramiplilate se trouve sous la forme de leur sels pharmaceutiquement acceptables ou tout autre dérivé du ramiprilate susceptible de libérer le ramiprilate dans l'organisme auquel le principe actif est administré.

## Revendications

1. Un inhibiteur de l'enzyme de conversion de l'angiotensine 1 en angiotensine 2, pour son utilisation dans un médicament ophtalmologique rétino-protecteur, ledit médicament étant destiné au traitement d'une affection ophtalmologique comportant une détérioration de la choroï-rétine, ladite affection étant choisie parmi :

   - les chorio-rétinopathies dégénératives du fort myope,
   - les chorio-rétinopathies séreuses centrales et épithéliopathies rétiniennes diffuses,
   - les dystrophies héréditaires de la rétine,
   - la diminution naturelle de l'acuité et/ou du champ visuel, ledit inhibiteur étant le ramipril, le ramiprilate, ou l'un de leurs sels pharmaceutiquement acceptables.

2. Un inhibiteur selon l'une quelconque des revendications précédentes, **caractérisé en ce que** ledit médicament comprend un véhicule pharmaceutiquement acceptable permettant l'administration dudit médicament sous forme orale, parentérale, intraveineuse, intramusculaire, transdermale ou topique.

3. Un inhibiteur selon l'une quelconque des revendications précédentes, **caractérisé en ce que** ledit médicament est à administration topique.

4. Un inhibiteur selon l'une quelconque des revendications précédentes, **caractérisé en ce que** ledit médicament est un collyre.

5. Un inhibiteur selon l'une quelconque des revendications 1 à 4, **caractérisé en ce que** ledit médicament est destiné au traitement des chorio-rétinopathies dégénératives du fort myope.

6. Un inhibiteur selon l'une quelconque des revendications 1 à 4, **caractérisé en ce que** ledit médicament est destiné au traitement de chorio-rétinopathies séreuses centrales et épithéliopathies rétiniennes diffuses.

7. Un inhibiteur selon l'une quelconque des revendications 1 à 4, **caractérisé en ce que** ledit médicament est destiné au traitement des dystrophies héréditaires de la rétine.

8. Un inhibiteur selon la revendication 7, **caractérisé en ce** ladite dystrophie héréditaire de la rétine est une rétinite pigmentaire.

9. Un inhibiteur selon l'une quelconque des revendications 1 à 4, **caractérisé en ce que** ledit médicament est destiné à prévenir ou ralentir, voire supprimer, une diminution naturelle de l'acuité visuelle et/ou du champ visuel.

10. Un inhibiteur selon l'une quelconque des revendications 1 à 4, et 9, **caractérisé en ce que** ledit médicament est destiné à prévenir ou inverser le déclin visuel chez un sujet vieillissant.

11. Un inhibiteur selon l'une quelconque des revendications précédentes, **caractérisé en ce que** ledit médicament est destiné à maintenir ou améliorer l'acuité visuelle et le champ visuel.

12. Un inhibiteur selon l'une quelconque des revendications précédentes, **caractérisé en ce que** ledit médicament est destiné au traitement d'une affection ophtalmologique qui comporte un facteur vasculaire.

13. Un inhibiteur selon l'une quelconque des revendications précédentes, **caractérisé en ce que** ledit médicament est destiné à un humain.

14. Médicament comprenant un inhibiteur de l'enzyme de conversion de l'angiotensine 1 en angiotensine 2, ledit inhibiteur étant le ramipril, le ramiprilate, ou l'un de leurs sels pharmaceutiquement acceptables, pour son utilisation comme médicament opthalmologique rétino-protecteur, tel que défini à l'une quelconque des revendications 1 à 13.

15. Utilisation d'un inhibiteur de l'enzyme de conversion de l'angiotensine 1 en angiotensine 2, ledit inhibiteur étant le ramipril, le ramiprilate, ou l'un de leurs sels pharmaceutiquement acceptables, dans la fabrication d'un médicament ophtalmologique rétinoprotecteur, tel que défini à l'une quelconque des revendications 1 à 13.

## Claims

1. Inhibitor of the enzyme for converting angiotensin 1 to angiotensin 2, for its use in a retinoprotective ophthalmologic medicament, said medicament being for use in the treatment of an ophthalmologic condition comprising a deterioration of the chorioretina, said condition being chosen from:

   - degenerative chorioretinopathies in severe myopia,
   - central serous chorioretinopathies and diffuse retinal epitheliopathies,
   - hereditary dystrophies of the retina,
   - the natural reduction in acuity and/or in visual field,

   said inhibitor being ramipril or ramiprilat, or a pharmaceutically acceptable salt thereof.

2. Inhibitor according to the preceding claim, **characterized in that** said medicament comprises a pharmaceutically acceptable carrier for the administration of said medicament in oral, parenteral, intravenous, intramuscular, transdermal or topical form.

3. Inhibitor according to either one of the preceding claims, **characterized in that** said medicament is for topical administration.

4. Inhibitor according to any one of the preceding claims, **characterized in that** said medicament is an eye lotion.

5. Inhibitor according to any one of Claims 1 to 4, **characterized in that** said medicament is for use in the treatment of degenerative chorioretinopathies in severe myopia.

6. Inhibitor according to any one of Claims 1 to 4, **characterized in that** said medicament is for use in the treatment of central serous chorioretinopathies and diffuse retinal epitheliopathies.

7. Inhibitor according to any one of Claims 1 to 4, **characterized in that** said medicament is for use in the treatment of hereditary dystrophies of the retina.

8. Inhibitor according to Claim 7, **characterized in that** said hereditary dystrophy of the retina is retinitis pigmentosa.

9. Inhibitor according to any one of Claims 1 to 4, **characterized in that** said medicament is for use in preventing or slowing down, or even stopping, a natural reduction in visual acuity and/or visual field.

10. Inhibitor according to any one of Claims 1 to 4 and 9, **characterized in that** said medicament is for use in preventing or reversing visual decline in an ageing individual.

11. Inhibitor according to any one of the preceding claims, **characterized in that** said medicament is for use in maintaining or improving visual acuity and visual field.

12. Inhibitor according to any one of the preceding claims, **characterized in that** said medicament is for use in the treatment of an ophthalmologic condition which comprises a vascular factor.

13. Inhibitor according to any one of the preceding claims, **characterized in that** said medicament is for a human being.

14. Medicament comprising an inhibitor of the enzyme for converting angiotensin 1 to angiotensin 2, said inhibitor being ramipril or ramiprilat, or a pharmaceutically acceptable salt thereof, for its use as a retinoprotective ophthalmologic medicament, as defined in any one of Claims 1 to 13.

15. Use of an inhibitor of the enzyme for converting angiotensin 1 to angiotensin 2, said inhibitor being ramipril or ramiprilat, or a pharmaceutically acceptable salt thereof, in the manufacture of a retinoprotective ophthalmologic medicament, as defined in any one of Claims 1 to 13.

## Patentansprüche

1. Inhibitor des Enzyms zur Umwandlung von Angiotensin-1 in Angiotensin-2 zur Verwendung in einem retinaprotektivem ophtalmologischen Medikament, wobei das Medikament zur Behandlung eines ophtalmologischen Leidens, umfassend eine Schädigung der Netzhaut-Aderhaut, bestimmt ist und das Leiden ausgewählt ist aus:

   - degenerativen Chorioretinopathien bei starker Kurzsichtigkeit,
   - zentrale, seröse Chorioretinopathien und diffuse Netzhautepitheliopathien,
   - erbliche Dystrophien der Retina,
   - natürliche Verminderung der Sehschärfe und/oder des Gesichtsfeldes,

   wobei der Inhibitor Ramipril, Ramiprilat oder eines ihrer pharmazeutisch annehmbaren Salze ist.

2. Inhibitor gemäß Anspruch 1, **dadurch gekennzeichnet, dass** das Medikament einen pharmazeutisch annehmbaren Träger umfasst, welcher die Verabreichung des Medikamentes in oraler, parenteraler, intravenöser, intramuskulärer, transdermaler oder topischer Form erlaubt.

3. Inhibitor nach einem der vorhergehenden Ansprüche, **dadurch gekennzeichnet, dass** das Medikament topisch verabreicht wird.

4. Inhibitor gemäß einem der vorhergehenden Ansprüche, **dadurch gekennzeichnet, dass** das Medikament in Form von Augentropfen vorliegt.

5. Inhibitor gemäß einem der Ansprüche 1 bis 4, **dadurch gekennzeichnet, dass** das Medikament zur Behandlung degenerativer Chorioretinopathien von starker Kurzsichtigkeit bestimmt ist.

6. Inhibitor gemäß einem der Ansprüche 1 bis 4 **dadurch gekennzeichnet, dass** das Medikament zur Behandlung von zentralen, serösen Chorioretinopathien und diffusen Netzhautepitheliopathien bestimmt ist.

7. Inhibitor gemäß einem der Ansprüche 1 bis 4, **dadurch gekennzeichnet, dass** das Medikament zur Behandlung erblicher Dystrophien der Netzhaut bestimmt ist.

8. Inhibitor gemäß Anspruch 7, **dadurch gekennzeichnet, dass** die erbliche Dystrophie der Netzhaut eine pigmentäre Netzhautentzündung ist.

9. Inhibitor gemäß einem der Ansprüche 1 bis 4, **dadurch gekennzeichnet, dass** das Medikament zur Verhinderung oder Verminderung, sogar Unterdrückung einer natürlichen Verminderung der Sehschärfe und/oder des Gesichtsfeldes dient.

10. Inhibitor gemäß einem der Ansprüche 1 bis 4 und 9, **dadurch gekennzeichnet, dass** das Medikament zur Verhinderung oder Umkehrung des Sehstärkeniederganges bei einem alternden Individuum bestimmt ist.

11. Inhibitor gemäß einem der vorhergehenden Ansprüche, **dadurch gekennzeichnet, dass** das Medikament zur Aufrechterhaltung und Verbesserung der Sehschärfe und des Gesichtsfeldes bestimmt ist.

12. Inhibitor gemäß einem der vorhergehenden Ansprüche, **dadurch gekennzeichnet, dass** das Medikament zur Behandlung eines ophtalmologischen Leidens bestimmt ist, das einen Gefäßfaktor umfasst.

13. Inhibitor gemäß einem der vorhergehenden Ansprüche, **dadurch gekennzeichnet, dass** das Medikament für Menschen bestimmt ist.

14. Medikament, umfassend einen Inhibitor des Enzyms zur Umwandlung von Angiotensin-1 in Angiotensin-2, wobei der Inhibitor Ramipril, Ramiprilat oder eines ihrer pharmazeutisch annehmbaren Salze ist, zur Verwendung als retinoprotektives, ophthalmologisches Medikament, wie in einem der Ansprüche 1 bis 13 definiert.

15. Verwendung eines Inhibitors des Enzyms zur Umwandlung von Angiotensin-1 in Angiotensin-2, wobei der Inhibitor Ramipril, Ramiprilat oder eines ihrer pharmazeutisch annehmbaren Salze ist, zur Herstellung eines retinoprotektiven, ophthalmologischen Medikaments, wie in einem der Ansprüche 1 bis 13 definiert.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0220107 A
- EP 0158157 A
- EP 0114333 A
- JP 05306296 B


### Littérature non-brevet citée dans la description


- **M. DÉTRY MOREL.** Perspectives dans le traitement médical de la neuropathie glaucomateuse - Bases de la neuroprotection. *J. Fr. Ophtalmol.,* 1999, vol. 22 (1), 122-134
- **SHAH et al.** *indian journal of pharmacology,* 1999, vol. 31, 110-115
- **KUSAKA-NAKAMURA et al.** *Acta Physiologica Hungarica,* 1988, vol. 71 (2), 251-269
- **GARG et al.** *Ann. Ophthalmol.,* 1998, vol. 30 (1), 33-37
- **KULSHRESTHA et al.** *IOVS,* Mars 1999, vol. 40 (4), 1652-B560
- **YAMAGAMI et al.** *Folia Ophthalmologica,* 1983, vol. 34 (2), 290-297
- Système rénine-angiotensine et ramipril, un nouvel inhibiteur de l'enzyme de conversion. **D. VASMANT ; N. BENDER.** journal of cardiovascular Pathology. Raven Press, 1989, vol. 14, S49-S56
- **BÜNNING P.** Inhibition of angiotensin converting enzyme by 2-(N-((S)-1-carboxy-3-phenoxy)-L-alanyl)-(1S,3S,5S)-2-azabicyclo (3.3.0)octane-3-carboxylic acid (HOE A98 diacid. Comparison with captopril and enalaprilat. *Drug Research,* 1984, vol. 34, 1406-1410
- **SHAPIRO R.** Activation and inactivation of rabbit pulmonary angiotensin converting enzyme. *Ph.D Thesis,* 1983